# EUROPÄISCHE PATENTSCHRIFT

(11) **EP 3 870 117 B1**
(45) Veröffentlichungstag und Bekanntmachung des Hinweises auf die Patenterteilung: **23.11.2022**
(21) Anmeldenummer: 19794500.9
(22) Anmeldetag: 23.10.2019
(51) Int. Cl.: A61F 2/72

(54) **VERFAHREN ZUM EINRICHTEN EINER STEUERUNG EINER ORTHOPÄDIETECHNISCHEN EINRICHTUNG SOWIE SYSTEM ZUR DURCHFÜHRUNG DES VERFAHRENS**
METHOD FOR SETTING UP A CONTROL OF AN ORTHOPEDIC TECHNICAL DEVICE AND SYSTEM FOR IMPLEMENTING THE METHOD
PROCÉDÉ DE CONFIGURATION D'UNE COMMANDE D'UN DISPOSITIF TECHNIQUE ORTHOPÉDIQUE ET SYSTÈME DE MISE EN OEUVRE DU PROCÉDÉ

(30) Priorität: 26.10.2018 DE 102018126788
(43) Veröffentlichungstag der Anmeldung: 01.09.2021
(73) Patentinhaber: Otto Bock Healthcare Products GmbH, 1110 Wien (AT)
(72) Erfinder: GÖBEL, Peter, 1070 Wien (AT); WALTER, Günther, 2331 Vösendorf (AT); AMSÜSS, Sebastian, 1180 Wien (AT); SCHACHINGER, Markus, 1220 Wien (AT); GERGER, Sigrid, 1150 Wien (AT)
(74) Vertreter: Gramm, Lins & Partner Patent- und Rechtsanwälte PartGmbB
(86) Internationale Anmeldenummer: PCT/EP2019/078897
(87) Internationale Veröffentlichungsnummer: WO 2020/083995

(56) Entgegenhaltungen:
- WO-A2-02/49534
- DE-A1-102009 030 217
- YOSHIZAKI HARA ET AL: "Development of an evaluation method for controlling skill of a myoelectric control hand", ELECTRONICS AND COMMUNICATIONS IN JAPAN, SCRIPTA TECHNICA. NEW YORK, US, Bd. 93, Nr. 5, 1. Mai 2010 (2010-05-01), Seiten 15-23, XP001555464, DOI: 10.1002/ECJ.10255 [gefunden am 2010-04-12]
- AKAZAWA K ET AL: "Technological developments in Japan - Compliant grasp in a myoelectric hand prosthesis - Controlling flexion angle and compliance with electromyogram signals", IEEE ENGINEERING IN MEDICINE AND BIOLOGY MAGAZINE, IEEE SERVICE CENTER, PISACATAWAY, NJ, US, Bd. 24, Nr. 4, 1. Juli 2005 (2005-07-01), Seiten 48-56, XP011136048, ISSN: 0739-5175
- HUDGINS B ET AL: "A NEW STRATEGY FOR MULTIFUNCTION MYOELECTRIC CONTROL", IEEE TRANSACTIONS ON BIOMEDICAL ENGINEERING, IEEE SERVICE CENTER, PISCATAWAY, NJ, USA, Bd. 40, Nr. 1, 1. Januar 1993 (1993-01-01) , Seiten 82-94, XP000354597, ISSN: 0018-9294, DOI: 10.1109/10.204774

## Beschreibung

Die Erfindung betrifft ein Verfahren zum Einrichten einer Steuerung einer orthopädietechnischen Einrichtung mit zumindest einem motorischen Antrieb, wobei die orthopädietechnische Einrichtung an einem Körperteil eines Patienten angelegt und mit Sensoren verbunden ist, die Steuersignale eines Patienten aufnehmen. Die Erfindung betrifft ebenfalls ein System zur Durchführung eines solchen Verfahrens.

Prothesen dienen dazu, die Form und/oder Funktion einer nicht vorhandenen Gliedmaße oder eines Körperteils zu ersetzen. Neben Prothesen, die allein die Form der nicht mehr vorhandenen Gliedmaße oder des nicht mehr vorhandenen Körperteils ersetzen, existieren Prothesen, die eine Funktionalität aufweisen. Häufig soll eine Prothese sowohl die Form als auch die Funktionalität möglichst naturgetreu ersetzen.

Prothesen, die eine Funktion ersetzen, sind beispielsweise Greifeinrichtungen, mit denen Gegenstände gegriffen und gehalten werden können. Die Betätigung einer solchen Greifeinrichtung, die beispielsweise als ein sogenannter Haken ausgebildet sein kann, kann über einen Seilzugmechanismus oder motorisch angetrieben verwirklicht werden. Es existieren motorisch angetriebene Prothesenhände, mit sowohl in Form als auch in Funktion einer natürlichen Hand nachgebildeten Fingern, die über myoelektrische Signale gesteuert werden, so dass unterschiedliche Greifbewegungen sowie Orientierungen der Prothesenhand in Abhängigkeit von myoelektrischen Signalen eingenommen werden können.

Prothesen für untere Extremitäten können als passive Prothesen ausgebildet sein, die über keine Verstelleinrichtungen verfügen. Dämpfer, die Bewegungen zwischen zwei gelenkig miteinander verbundene Komponenten beeinflussen, können vorgesehen sein, eine Verstellung ist jedoch nicht vorgesehen. Darüber hinaus gibt es Prothesen für untere Extremitäten, bei denen Dämpfereinstellungen über motorische Antriebe in Abhängigkeit von erfassten Sensordaten, beispielsweise Beschleunigungen, Kräfte oder Winkel verstellt werden können. Ebenfalls sind aktive Prothesen bekannt, die Motoren aufweisen, die eine Flexion und/oder Extension unterstützen oder alleine bewirken. Diese Motoren werden über eine Steuerung aktiviert oder deaktiviert. Eine myoelektrische Steuerung, bei der Muskelkontraktionen aufgenommen und als Steuersignale verwendet werden, ist ebenfalls bekannt.

Neben Prothesen sind entsprechende Orthesen bekannt, die an vorhandene Gliedmaßen oder Körperteile angelegt werden und die Bewegungen beeinflussen, unterstützen oder Zuordnungen von Körperteilen beeinflussen oder aufrechterhalten. Auch Orthesen können mit Bremsen, Dämpfern oder motorischen Antrieben versehen sein, um Widerstände zu verändern oder Bewegungen zu unterstützen oder auszuführen.

Um die orthopädietechnischen Einrichtungen wie Orthese oder Prothese steuern zu können, werden Sensoren eingesetzt, die Signale erfassen, die durch den Nutzer der orthopädietechnischen Einrichtung oder die Benutzung der orthopädietechnischen Einrichtung erzeugt werden. Dies können eine Vielzahl von Signalen sein, beispielsweise myoelektrische Signale oder Zustands-, Belastung- und/oder Bewegungssignale. Um die orthopädietechnische Einrichtung über myoelektrische Signale passend steuern zu können, müssen die Patienten oder Nutzer der orthopädietechnischen Einrichtung bestimmte Kontraktionsmuster erlernen, die den jeweiligen Funktionen zugeordnet sind. Üblicherweise ist in der jeweiligen Steuerung der orthopädietechnischen Einrichtung, beispielsweise der Orthese oder Prothese, ein Programm abgelegt, in dem eine bestimmte Abfolge von Steuerungssignalen mit unterschiedlichen Anzahlen an Signalimpulsen, unterschiedlichen Signalstärken und/oder unterschiedliche Signaldauern festgelegt ist. Durch beispielsweise mehrfaches Anspannen eines bestimmten Muskels oder eines bestimmten Restmuskels in einer bestimmten Frequenz mit einer bestimmten Intensität wird erkannt, dass ein bestimmtes Programm ausgeführt werden soll. Daraufhin wird ein Antrieb aktiviert, beispielsweise um eine Dämpfereinrichtung hinsichtlich des Widerstandes zu verändern oder um zwei Komponenten einer orthopädietechnischen Einrichtung zueinander zu verlagern. Beispielsweise kann bei einer Prothesenhand das Öffnen oder Schließen der Hand, das Drehen des Handgelenks oder eine Extension oder Flexion der Prothesenhand ausgeführt werden. Bei einem angetriebenen Prothesenfuß können Plantarflexion und Dorsalflexion bewirkt oder unterstützt werden. Insbesondere bei orthopädietechnischen Einrichtungen mit einer Vielzahl von Funktionen wie einer Prothesenhand kann es schwierig sein, die jeweiligen Signalmuster zu erlernen. Darüber hinaus kann es aufgrund von individuellen Eigenschaften des Nutzers nicht oder nur eingeschränkt möglich sein, bestimmte Signalfolgen zu erzeugen.

In dem Artikel "Development of an evaluation method for controlling skill of a myoelectric control hand", Yoshizaki et al, Electronics and Communications in Japan, Vol. 93, No. 5, 2010, betrifft eine Studie zur Entwicklung einer neuen Methode zur Bewertung der Steuerungsfähigkeiten einer myoelektrischen gesteuerten Prothesenhand. An dieser Studie nahmen vier Amputierte teil. Es wurden zwei Signale gemessen, die zur Steuerung der Bewegung der Prothesenhand benötigt werden. Die beiden Signale wurden als die durchschnittlichen gleichgerichteten Elektromyogramme der Unterarmstrecker und -beuger zugeordnet. Die Werte der Signale stiegen in Abhängigkeit von dem Muskelkontraktionsniveau. Es wurde ein Therapie zur Verwendung der Prothesenhand durchgeführt, um die Fähigkeit zu verbessern, getrennte Kontraktionen zweier Muskeln zu erzeugen. Die Fähigkeit in jeder Bewegung wurde durch den Regressionskoeffizienten bewertet, der nach der Methode der kleinsten Quadrate berechnet wurde.

Die WO 02/49534 A2 betrifft ein Verfahren zum Trainieren eines Prothesenträgers. Hierzu wird dieser mit Sensoren versorgt und soll mit diesen eine auf einem Bildschirm dargestellte virtuelle Prothese steuern und dies hierdurch trainieren. Die Vorrichtung dient dazu, die Präzision der Steuerung über vorgegebene Signalfolgen der Muskelkontraktion zu trainieren, ohne dass eine Prothese angelegt werden muss.

Aufgabe der vorliegenden Erfindung ist es daher, ein Verfahren und ein System zur Durchführung des Verfahrens bereitzustellen, mit dem eine vereinfachte Anpassung an den jeweiligen Nutzer erfolgen kann.

Erfindungsgemäß wird diese Aufgabe durch ein Verfahren mit den Merkmalen des Hauptanspruches und ein System mit den Merkmalen des nebengeordneten Anspruchs gelöst. Vorteilhafte Ausgestaltungen und Weiterbildungen der Erfindung sind in den jeweiligen Unteransprüchen, der Beschreibung sowie den Figuren offenbart.

Das Verfahren zum Einrichten einer Steuerung einer orthopädietechnischen Einrichtung mit zumindest einem motorischen Antrieb, die an einem Körperteil eines Patienten angelegt und mit Sensoren verbunden ist, die Steuersignale des Patienten aufnehmen, sieht das Ausgeben einer optischen, akustischen und/oder taktilen Darstellung einer Betätigung einer Gliedmaße als Aufforderung an den Patienten vor, diese Betätigung auszuführen. Anschließend werden Steuersignale erfasst, die von dem Patienten nach dessen Aufforderung als willkürliche Reaktion erzeugt werden. Die erfassten Steuersignale werden der ausgeführten Betätigung zugeordnet und damit als Steuersignale identifiziert, die als Umschaltsignale dienen können. Darüber hinaus werden diese erfassten Steuersignale einer Funktion zugeordnet, in der der zumindest eine motorische Antrieb aktiviert, deaktiviert oder in seiner Drehrichtung umgekehrt wird, so dass eine Verbindung zwischen den erfassten Steuersignalen zu der auszuführenden Funktion, z.B. "Handgelenksflexion" oder "Handgelenksextension" der orthopädietechnischen Einrichtung eingerichtet wird. Nach dem Zuordnen der erfassten Steuersignale zu der jeweiligen Funktion werden die erfassten Steuersignale und/oder die Funktion ausgegeben, damit die bevorzugt automatisierte Zuordnung aufgrund einer standardisierten Voreinstellung erfasst werden kann. Die Ausgabe ermöglicht eine Überprüfung der automatisierten Berechnung der Einstellparameter oder der ausgewählten Funktion, so dass eine Anpassung an das individuelle Versorgungsoptimum der Nutzer der orthopädietechnischen Einrichtung möglich wird. Darüber hinaus wird ein Feedback für den Nutzer gegeben und die jeweiligen Steuersignale dokumentiert, so dass über den Verlauf der Nutzung der orthopädietechnischen Einrichtung, beispielsweise Prothese oder Orthese, Aussagen über eine Verbesserung oder eine Verschlechterung des jeweiligen Zustandes erfolgen kann. Mit dem vorgeschlagenen Verfahren werden die jeweiligen Steuersignale ausgewertet und hinsichtlich ihrer Eignung zur Auswahl einer entsprechenden Funktion überprüft. Dies geschieht automatisch, so dass die Einstellung der Parameter wie Signalabfolge, Signaldauer und Signalintensität nicht mehr von den Erfahrungswerten eines Orthopädietechnikers abhängig ist, sondern aufgrund einer automatisierten Analyse der Steuerungssignale erfolgt. Die Funktionsauswahl ist dabei getrennt von der Ausführung der Funktion zu sehen. Die Ausführung der Funktion wird durch ein anderes Signal ausgelöst. Das sogenannte Aktivierungssignal aktiviert oder deaktiviert den Antrieb in der ihm zugeordneten Funktion in einer Art und Weise, wie sie in der Steuerung abgelegt ist. Wird ein Aktivierungssignal von dem Patienten erzeugt, während die erste Funktion durch das entsprechende Steuersignal oder das Umschaltsignal ausgewählt wurde, wird beispielsweise ein Handgelenksflexionsantrieb solang aktiviert, bis das Aktivierungssignal nicht mehr erzeugt wird. Wird dann mit einem Umschaltsignal eine andere Funktion ausgewählt, beispielsweise die Handgelenksrotation, wird durch das Aktivierungssignal der Antrieb für die Handgelenksrotation solange und so schnell aktiviert, wie das Aktivierungssignal anliegt. Somit muss der Patient nur ein Aktivierungssignal erzeugen, das nach der Auswahl der jeweils auszuführenden Funktion durch das Steuersignal den jeweiligen Antrieb aktiviert, deaktiviert oder in seiner Drehrichtung umkehrt. Die Funktion bestimmt, was ausgeführt werden kann, das Aktivierungssignal bestimmt, wie die Funktion ausgeführt wird, das Steuersignal schaltet zwischen den zur Verfügung stehenden Funktion um. Wie die Umschaltung am besten stattfindet und eingerichtet wird, ist ein Aspekt der Erfindung.

Über die Ausgabe der Steuersignale an den Nutzer oder einen Orthopädietechniker ist es möglich, Verbesserungsmöglichkeiten für den Nutzer bei der Erzeugung der Steuerungssignale aufzuzeigen und somit eine Rückmeldung zu geben, ob die Steuerungssignale überhaupt geeignet sind, ob sich die Qualität der Signale verändert hat und ob sie gegenüber vorherigen Messungen oder Erfassungszeiträumen verbessert oder verschlechtert hat.

In einer Weiterbildung des Verfahrens ist es vorgesehen, dass die Steuersignale über genau zwei Elektroden oder ein Elektrodenpaare aufgenommen werden. Neben der Möglichkeit, zumindest ein Steuersignal, insbesondere ein myoelektrisches Signal, über mindestens eine Elektrode aufzunehmen, ist vorgesehen, dass zwei oder mehr Elektrodenpaare zur Aufnahme der Steuersignale vorhanden sind, dann erfolgt bei der Aufnahme der Steuersignale über Elektrodenpaare eine Signalerfassung über zwei oder mehr Kanäle, so dass Umschaltsignale einfach erzeugt werden können. Die beiden Elektrodenpaare oder Sensorenpaare erzeugen Umschaltsignale, die dazu dienen, den Antrieb zu aktivieren, deaktivieren oder in der Richtung des Tätigwerdens umzukehren. Über die Erfassung der Steuersignale über genau zwei Elektroden oder ein Elektrodenpaare verringert sich die Komplexität der Signalaufnahme, das Erzeugen der Steuersignale wird vereinfacht, insbesondere das Erzeugen von myoelektrischen Signalen, da nur begrenzte Muskelgruppen aktiviert werden müssen. Bei einer Anordnung an antagonistisch wirkenden Muskeln kann eine hohe Signalqualität der jeweiligen Umschaltsignale erreicht werden, auch bei der Verwendung von zwei Elektrodenpaaren.

Eine Weiterbildung sieht vor, dass aus den erfassten Steuersignalen Parameter abgeleitet werden, die für die Zuordnung der jeweiligen Funktion zu den Steuersignalen relevant sind. Diese Parameter sind beispielsweise die Signallänge, der Signalort oder der Kanal, auf dem das Signal erzeugt oder aufgenommen wird, die Anzahl von Signalen, die Frequenz von Signalen und Amplitude von Signalen, insbesondere, wenn myoelektrische Signale erfasst werden. Grundsätzlich handelt es sich bei den Steuersignalen um Signale, die von dem Menschen willkürlich erzeugt werden. Neben myoelektrischen Signalen können dies auch Drucksignale oder Signale von Drucksensoren oder optische Signale oder mit einem Blutfluss korrelierte Signale sein.

Eine Weiterbildung der Erfindung sieht vor, dass die Bewegungsaufforderung in Form vorgeschlagener Schaltsignale ausgegeben wird. Als Schaltsignale können beispielsweise eine vorgegebene Anzahl oder ein vorgegebenes Muster an Muskelkontraktionen vorgesehen sein, aus denen die Parameter für die Steuersignale ermittelt werden. Die Schaltsignale sind beispielsweise Impulssteigung, Impulshöhe, Impulsdauer von Kontraktionen und daraus abgeleitete myoelektrische Signale.

Eine Weiterbildung des Verfahrens sieht vor, dass eine Zuordnung eines Steuersignals zu einer Funktion auf der Grundlage einer Signalstärke nach Überschreiten eines festgelegten Schwellwertes erfolgt. Durch das Festlegen eines Schwellwertes wird verhindert, dass durch nicht beabsichtigte Aktionen, beispielsweise durch unwillkürliche Bewegungen oder Kokontraktionen, Steuersignale erzeugt und als Signale zur Auslösung einer Funktion angesehen oder interpretiert werden. Nur wenn das erfasste Signal eine gewisse Qualität hat, z.B. eine gewisse Intensität, Impulssteigung, Impulshöhe oder Impulsdauer, wird dieses als ein Trigger-Signal verwendet, anderenfalls wird ein solches Signal nicht zur Einleitung einer Funktion verwendet.

Vor der Zuordnung der Steuersignale zu einer Funktion kann eine Bestätigungsabfrage durchgeführt werden, um die vorgeschlagene oder automatische Zuordnung des Steuersignals zu einer Funktion zu bestätigen oder zu korrigieren. Die Bestätigungsabfrage wird automatisch gestellt, eine Bestätigung erfolgt dann durch den Orthopädietechniker oder den Nutzer der orthopädietechnischen Einrichtung.

In einer Weiterbildung wird vor der Zuordnung zu einer Funktion oder einem Umschaltvorgang zu einer anderen Funktion eine Auswertung der Steuersignale hinsichtlich der Signalqualität durchgeführt. Die Signalqualität besteht insbesondere in der Dauer des Signals, der Intensität oder Stärke des Signals sowie in der Wiederholbarkeit. Es können mehrere Aufforderungen an den Patienten oder Nutzer der orthopädietechnischen Einrichtung erfolgen, ein und dieselbe Betätigung auszuführen, beispielsweise ein Bein zu strecken oder eine Hand zu schließen. Wird das durch den Patienten erzeugte und von den Sensoren erfasste Steuersignal nicht über alle Wiederholungen in einer ausreichenden Qualität oder einer ausreichenden Identifizierbarkeit ausgegeben, liegt keine ausreichende Wiederholbarkeit vor, so dass keine Zuordnung des Steuersignals als Umschaltsignal zu der jeweiligen Funktion oder als Aktivierungssignal stattfinden kann. Bei nicht ausreichender Signalqualität wird eine Fehlermeldung oder ein Korrekturvorschlag ausgegeben, wobei der Korrekturvorschlag als Rückmeldung auch als Trainingsaufforderung eingesetzt werden kann. Durch die Rückmeldung oder den Korrekturvorschlag wird der Patient oder der Nutzer der orthopädietechnischen Einrichtung angehalten, eine Vielzahl beispielsweise von Muskelkontraktionen mit einer gleichbleibenden Intensität, Dauer und/oder Geschwindigkeit durchzuführen, um die gewünschte Signalqualität zu erreichen. Dadurch kann verhindert werden, dass über die Zeit, beispielsweise die Muskeltätigkeit nachlässt und die Kontraktionsfähigkeit, Kontraktionsstärke und Kontraktionsgeschwindigkeit sowie gegebenenfalls Kontraktionsdauer bei einer bestimmten Kontraktionsstärke nachlässt. Durch den Korrekturvorschlag, beispielsweise die Intensität der Kontraktion zu erhöhen, um die einmal festgelegte Signalqualität zu erreichen, kann eine Muskelatrophie verhindert oder verlangsamt werden.

Die Steuersignale können vor der Zuordnung zu den jeweiligen Funktionen oder dem Umschaltvorgang zu einer Funktion mit festgelegten oder errechneten Korrekturfaktoren beaufschlagt werden. Die Korrekturfaktoren können Schwellwerte, Zeiten oder Verstärkungsfaktoren betreffen, so dass die aufgenommenen und erfassten Signale aufbereitet, gefiltert oder auf andere Art und Weise elektronisch verändert werden, damit sie besser für die Identifikation und Auslösung von Funktionen geeignet sind.

Die Steuersignale, die von dem Patienten nach der Aufforderung als willkürliche Reaktion erzeugt werden, werden in einer Weiterbildung nach jeder Aufforderung gespeichert, um diese entweder einer Mittelwertbildung zugänglich zu machen oder um diese über die Zeit auswerten zu können. Anhand der gespeicherten Steuersignale nach jeder Aufforderung kann eine Entwicklung von Antworten auf Aufforderungen nachverfolgt werden, so dass beispielsweise Veränderungen in der Impulshöhe, Impulsdauer oder Impulssteigung erfasst und nachvollzogen werden kann.

In einer Weiterbildung werden die Steuersignale, die von dem Patienten nach Aufforderung als willkürliche Reaktion erzeugt werden, mit vorgegebenen Zielwerten verglichen und hinsichtlich des Erreichens dieser Zielwerte bewertet. Werden die vorgegebenen Zielwerte erreicht, wird ein positives Bestätigungssignal ausgegeben oder das Erreichen in einer entsprechenden Datei vermerkt. Wird ein vorgegebener Zielwert nicht erreicht oder nur knapp erreicht, wird eine Fehlermeldung oder Warnmeldung ausgegeben, um den Patienten darüber zu informieren, dass das erzeugte Steuersignal nicht oder beinahe nicht den Anforderungen zum Auslösen einer Funktion genügt.

Das System zur Durchführung eines Verfahrens, wie es oben beschrieben worden ist, sieht eine an ein Körperteil eines Patienten anlegbare orthopädietechnische Einrichtung mit zumindest einem motorischen Antrieb vor, mit einer Ausgabeeinrichtung, die optische, akustische und/oder taktile Darstellungen einer Betätigung einer Gliedmaße als Aufforderungen an den Patienten, diese Betätigung auszuführen, ausgibt, mit Sensoren, die mit der orthopädietechnischen Einrichtung verbunden und an dem Patienten festlegbar sind und Steuersignale des Patienten aufnehmen. Das System weist weiterhin eine elektronische Auswerteeinrichtung auf, in der die Steuersignale, die von dem Patienten nach Aufforderung als willkürliche Reaktion erzeugt werden, verarbeitet, ausgewertet und einer Funktion zugeordnet werden. Die Auswerteeinrichtung ist insbesondere als ein Computer oder eine Datenverarbeitungseinrichtung mit einem Speicher und gegebenenfalls einer Energieversorgung sowie einer Ausgabeeinrichtung versehen, um die Signale aufzunehmen, zu verarbeiten, zu speichern und auszugeben, entweder an einen motorischen Antrieb oder an eine Ausgabeeinrichtung, die entweder die Sensorsignale als solche oder ausgewertete und aufbearbeitete Sensorsignale ausgibt. Die Ausgabeeinrichtung gibt zudem die jeweilige, dem Steuersignal zugeordnete Funktion aus, so dass überprüft und ausgewertet werden kann, ob die durch die Auswerteeinrichtung vorgenommene Zuordnung des Steuersignals als Umschaltsignal zu der Funktion zutreffend ist, welche Qualität das Steuersignal hat, ob die dem Steuersignal zugeordnete Funktion die gewünschte oder am besten geeignete ist oder ob eine andere Funktion eher geeignet ist, die Funktion auszuwählen, um die orthopädietechnische Einrichtung damit zu betreiben.

Die Auswerteeinrichtung kann eine Schnittstelle aufweisen, über die die Zuordnung der Steuersignale zu der Funktion beeinflussbar ist, so dass eine von der Auswerteeinrichtung vorgenommene, automatische Zuordnung korrigiert, gelöscht oder ergänzt werden kann.

Nachfolgend werden Ausführungsbeispiele der Erfindung anhand der beigefügten Figuren näher erläutert. Es zeigen:
- Figur 1 -: eine schematische Darstellung einer angetriebenen orthopädietechnischen Einrichtung, die mit einem Computer verbunden ist;
- Figur 1a -: eine schematische Darstellung der Funktionsweise von Umschaltsignalen und Aktivierungssignalen;
- Figur 2 -: ein Ablaufdiagramm;
- Figur 3 -: eine Zuordnungsmatrix aus möglichen Triggersignalen und möglichen Funktionen;
- Figur 4 -: Signaldarstellungen vor und nach einer Verstärkung; sowie
- Figuren 5 - 8: - Bedingungen hinsichtlich der Signalqualität.

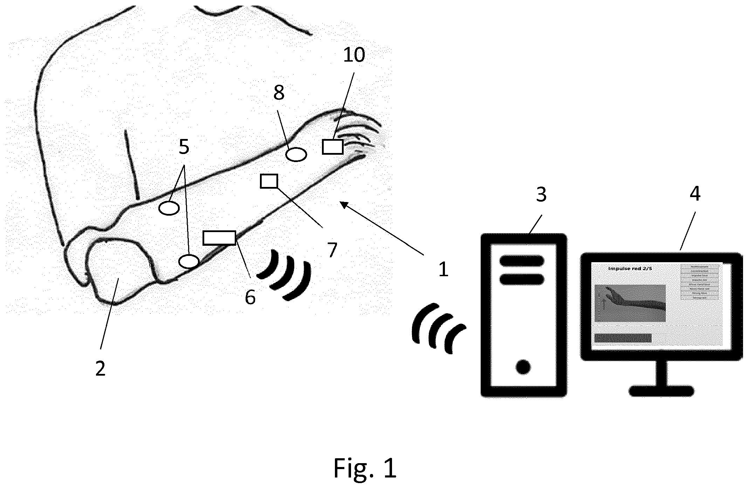

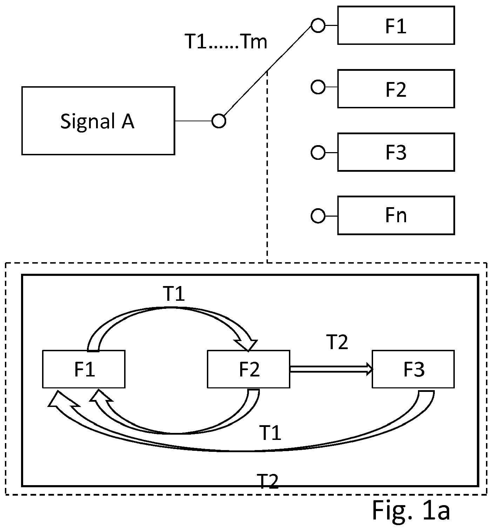

In der Figur 1 ist in einer schematischen Darstellung eine orthopädietechnische Einrichtung 1 in Gestalt einer Handprothese dargestellt. Die orthopädietechnische Einrichtung 1 weist einen Unterarmschaft auf, der an einem Unterarmstumpf an einem Arm 2 eines Patienten festgelegt ist. Alternativ zu einer Prothese kann die orthopädietechnische Einrichtung 1 auch als Orthese ausgebildet sein. Alternativ zu einer orthopädietechnischen Einrichtung an einer oberen Extremität kann diese auch an einer unteren Extremität festgelegt sein, beispielsweise als eine Beinorthese oder ein Prothesenbein. An der orthopädietechnischen Einrichtung 1 sind Sensoren 5 angeordnet, die Steuersignale erfassen, die von dem Patienten oder dem Nutzer der orthopädietechnischen Einrichtung 1 erzeugt werden. Dies können insbesondere Kontraktionssignale sein, also Signale, die ihre Basis in Muskelkontraktionen haben. Neben insbesondere myoelektrischen Signalen können dies auch direkte Nervenimpulse, Dichteänderungen, Temperaturänderungen, Durchflusswiderstände oder elektrische Widerstände sein.

In der orthopädietechnischen Einrichtung 1 ist zumindest ein motorischer Antrieb 10, insbesondere ein elektromotorischer Antrieb vorgesehen, der von einer Steuereinrichtung 7 angesteuert werden kann, um Komponenten der orthopädietechnischen Einrichtung 1 zu bewegen, beispielsweise um einzelne Finger zu bewegen, um die Prothesenhand um die Stumpflängsachse zu drehen, um eine Handgelenksflexion oder eine Handgelenksextension durchzuführen oder um andere Bewegungen einer motorischen angetriebenen Prothesenhand durchführen zu können. Entsprechend dem Aufbau der orthopädietechnischen Einrichtung können die Antriebe 10 separat voneinander angesteuert werden oder aber auch gemeinsam. Bei einer anderen Ausgestaltung der orthopädietechnischen Einrichtung 1, beispielsweise als eine Beinorthese, können die Antriebe 10 eine Flexion oder eine Extension eines Unterschenkelteils oder eines Fußteils bewirken.

Die orthopädietechnische Einrichtung 1 weist ebenfalls eine Energiespeichereinrichtung 8 auf, um den motorischen Antrieb 10 mit ausreichend Energie zu versorgen, beispielsweise mit elektrischer Energie. Die jeweiligen Funktionen oder Schalt- oder Umschaltvorgänge für den jeweiligen motorischen Antrieb 10 werden über die Steuereinrichtung 7 koordiniert und veranlasst. Die Steuereinrichtung 7 ist mit den Sensoren 5 verbunden, beispielsweise mit einem Elektrodenpaar, über die die Steuersignale aufgrund von Kokontraktionen im Unterarm aufgenommen werden. Der orthopädietechnischen Einrichtung 1 ist ebenfalls eine Sende- und Empfangseinrichtung 6 zugeordnet, mit der es möglich ist, Steuersignale, aufbereitete Steuersignale oder andere Informationen zu empfangen oder zu senden. Die aufgenommenen Steuersignale können beispielsweise an einen Computer 3 gesendet werden, in dem die aufgenommenen Steuersignale verarbeitet werden. Der Computer 3 ist mit einer Ausgabeeinrichtung 4 verbunden, die im dargestellten Ausführungsbeispiel ein Display ist. Die Ausgabeeinrichtung 4 kann zusätzlich zu einer optischen Darstellung auch eine akustische Darstellung und/oder eine taktile Darstellung ausgeben, beispielsweise über niedrig frequente Vibrationen oder aber durch Bewegungen von Modellen.

Um die orthopädietechnische Einrichtung 1 an den jeweiligen Patienten anzupassen, ist es notwendig, bestimmte Steuersignale, die von den Sensoren 5 erfasst werden und von dem Patienten initiiert werden, aufzunehmen und bestimmten Funktionen zuzuordnen, die in der Steuereinrichtung 7 abgelegt sind und mit Schaltvorgängen oder Umschaltvorgängen für die Antriebe 10 verbunden sind. Ein bestimmtes Kontraktionsmuster wird dabei beispielsweise als Umschaltsignal 1, Umschaltsignal 2, Umschaltsignal 3, etc. dem Schließen der Hand zugeordnet, ein anderes dem Öffnen, ein drittes einer Handgelenksrotation, ein weiteres der Handgelenksflexion, der Extension usw. Die Kontraktionsmuster werden nicht direkt in Bewegungen umgesetzt, sondern dienen als Umschaltsignale, um von eine Funktion A zu einer Funktion B zu gelangen. Ein üblicher Ansatz war bislang, dass ein Patient solange vorgegebene Kontraktionsmuster üben musste, bis die dadurch erzeugten Steuersignale den in der Steuereinrichtung 7 vorgegebenen Werten entsprechen. Dies ist insbesondere bei einer klassischen Versorgung mit einem Elektrodenpaar, also mit zwei Kanälen, außerordentlich schwierig und anstrengend für den Patienten. Eine Vielzahl von Kontraktionsmustern muss dauerhaft und stabil erzeugt werden können, die Kontraktionen müssen in der richtigen Reihenfolge, in der richtigen Stärke und über die richtige Zeit hinweg aufgebracht werden. Besonders schwierig ist das Umschalten von der einen Funktion in eine andere Funktion, was beispielsweise über ein weiteres Kontraktionsmuster eingeleitet wird. Mit der vorliegenden Erfindung wird erreicht, dass erstens Umschaltsignale, die der Anwender beispielsweise aufgrund einer Verletzung nicht ausführen kann, eliminiert werden und zweitens diejenigen Umschaltsignale, die von dem Anwender ausgeführt werden können, in ihren Parametern dergestalt optimiert werden, dass der Anwender sie leicht und wiederholbar ausführen kann. Die Auswahl der Parameter und die Optimierung der Parameter erfolgen bevorzugt automatisch und nicht auf der Basis eines nicht reproduzierbaren Gefühls oder der Erfahrung eines Orthopädietechnikers.

Um einem Patienten die Benutzung einer orthopädietechnischen Einrichtung 1 zu erleichtern, die Anpassung der jeweiligen orthopädietechnischen Einrichtung 1 an den Patienten zu verbessern und insbesondere das Einstellverfahren zu vereinfachen, wird dem Patienten über die Ausgabeeinrichtung 4 eine optische, akustische oder taktile Darstellung einer Betätigung einer Gliedmaße als Aufforderung ausgegeben, diese Betätigung auszuführen. In dem dargestellten Ausführungsbeispiel soll eine Handgelenksextension erfolgen. Nach Wahrnehmung der Ausgabe in der Ausgabeeinrichtung 4 führt der Patient diese von ihm gewollte Betätigung aus. Bei einer prothetischen Versorgung kann die Bewegung selbst natürlich nicht ausgeführt werden, es werden lediglich die dazu von dem Patienten natürlicherweise eingesetzten Muskeln aktiviert, es findet eine entsprechende Kontraktion der für diese Bewegung beteiligten Muskeln statt. Bei einer orthetischen Versorgung kann die Bewegung ggf. ausgeführt werden, eventuell jedoch nicht mit einer ausreichenden Kraft, so dass eine Unterstützung durch einen Antrieb 10 notwendig ist.

Über den Sensoren 5 oder das Elektrodenpaar werden Steuersignale erfasst, beispielsweise myoelektrische Signale aufgrund der Muskelkontraktion. Die erfassten Steuersignale werden zu der ausgeführten Betätigung zugeordnet, vorzugsweise automatisiert zugeordnet und über die Sende- und Empfangseinrichtung 6 dem Computer 3 vorzugsweise drahtlos übermittelt. Alternativ ist eine kabelgebundene Verbindung der orthopädietechnischen Einrichtung 1 mit dem Computer 3 vorgesehen. In dem Computer 3 werden die erfassten Steuersignale zu der vorgeführten und auf der Ausgabeeinrichtung 4 gezeigten Betätigung einer Funktion zugeordnet, in der der zumindest eine motorische Antrieb 10 aktiviert, deaktiviert oder in seiner Drehrichtung umgekehrt wird. Das der Handgelenksextension zugeordnete Steuersignalmuster wird der Funktion zugeordnet und in entsprechende Schaltbefehle für den jeweils notwendigen motorischen Antrieb 10 umgewandelt, um eine Handgelenksextension der orthopädietechnischen Einrichtung 1 durchführen zu können.

In der Figur 1a ist der grundsätzliche Unterschied zwischen dem Umschalten zwischen einzelnen Funktionen und der Aktivierung der jeweiligen Funktion dargestellt. Die obere Darstellung zeigt links das Signal A, das die jeweils ausgewählte oder geschaltete Funktion aktiviert. In der oberen Darstellung rechts sind die einzelnen Funktionen F1, F2 bis Fn dargestellt. Die Funktion F1, F2, F3 bis Fn legen beispielsweise fest, welche Bewegung ausgeführt werden kann, beispielsweise eine Innenrotation, eine Außenrotation, das Schließen einer Hand, das Öffnen einer Hand, die Extension eines Unterarms oder die Flexion. Um zwischen den einzelnen Funktionen F1 bis Fn umschalten zu können, damit nach einer Innenrotation einer Prothesenhand eine Außenrotation erfolgen kann oder aber damit nach einer Unterarmextension ein Zugreifen durch das Schließen einer Prothesenhand erfolgen kann, werden die Steuersignale einer Funktion zugeordnet und dienen als Umschaltsignale oder Triggersignale, die es ermöglichen, von der Funktion F1 zu der Funktion F2 oder F3 zu gelangen, damit überhaupt einen andere Bewegung ausgeführt werden kann. Die Steuersignale als Umschaltsignale oder Triggersignale lösen keine Betätigung der orthopädietechnischen Einrichtung oder eine Aktivierung oder Deaktivierung eines motorischen Antriebes aus, sondern geben nur vor, welche Funktion mit einem nachfolgenden Signal von dem Patienten, einem sogenannten Aktivierungssignal, ausgeführt werden kann. Mit dem Steuersignal in Gestalt des Triggersignals oder Umschaltsignals T1 wird beispielsweise die Funktion F1 mit der Möglichkeit eine Prothesenhand zu schließen ausgewählt. Diese Funktion F1 ist mit den Befehlen hinterlegt, welche Motoren oder welcher Motor in welcher Drehrichtung aktiviert wird, sobald ein Aktivierungssignal empfangen wird. Ein Triggersignal T2 kann die Funktion F2 mit der Tätigkeit des Handöffnens auswählen, wozu die Antriebsrichtung des Motors oder der Motoren umgedreht wird. Die übrigen Funktionen F3 bis Fn können entsprechend mit Aktionen hinterlegt sein und mit dem entsprechenden Triggersignal ausgewählt werden. Als Steuersignale oder Triggersignale können Muskelkontraktionen, Impulsfolgen, Impulsdauern und dergleichen verwendet werden. Ist die jeweilige Funktion ausgewählt, wird die damit verbundene Tätigkeit oder Bewegung durch das Aktivierungssignal A aktiviert. Dieses Signal A aktiviert die jeweils geschaltete Funktion und aktiviert oder deaktiviert den Antrieb oder reversiert die Drehrichtung, wobei dieses Signal A auch zu einer proportionalen Regelung des jeweiligen Antriebes dienen kann. Wird beispielsweise eine sich erhöhende Kontraktion festgestellt, kann die Tätigkeit in einer höheren Geschwindigkeit oder mit einer höheren Kraft ausgeführt werden, beispielsweise um einen Gegenstand schneller oder fester zu umgreifen.

In der unteren Darstellung der Figur 1a ist das Umschalten zwischen den einzelnen Funktionen F1, F2 und F3 beispielhaft dargestellt. Befindet man sich in der Funktion F1, kann durch das Steuersignal als Triggersignal T1 zur zweiten Funktion F2 geschaltet werden. Durch erneutes Ausüben des Steuersignals T1 wird zurück zur Ausgangsfunktion F1 geschaltet. Wird ausgehend von der Funktion F2 ein zweites, anderes Steuersignal T2 durch den Patienten erzeugt, wird zu der nächsten Funktion F3 geschaltet. Über ein erneutes Triggersignal T2 wird wieder zur Funktion F1 zurückgeschaltet. Alternative Umschaltregeln können vorhanden sein, beispielsweise kann durch ein erneutes Triggersignal T2 zur Funktion F2 zurückgeschaltet werden, von der aus durch erneutes Erzeugen des Triggersignals T1 zur Funktion F1 geschaltet werden kann.

Bevor die endgültige Zuordnung der erfassten Steuersignale zu der jeweiligen Funktion erfolgt, werden die erfassten Steuersignale über die Ausgabeeinrichtung ausgegeben. Alternativ oder zusätzlich kann die jeweilige Funktion nach der jeweiligen Zuordnung des Steuersignals als Trigger- oder Umschaltsignal zu dieser Funktion ausgegeben werden, um zu überprüfen, ob die Zuordnung vorteilhaft oder günstig ist. Über diese Ausgabe kann eine Rückmeldung hinsichtlich des aktuellen Aktivierungsstatus und der jeweiligen Zuordnung eines Steuersignals oder mehrerer Steuersignale zu einer jeweiligen Funktion erfolgen. Da die Zuordnung zunächst im Rahmen eines Standardprogrammes automatisiert vorgeschlagen worden ist, kann durch die Ausgabe eine individuelle Anpassung, beispielsweise durch einen Orthopädietechniker, erfolgen. Dadurch wird der Einstellvorgang von orthopädietechnischen Einrichtungen, insbesondere von Myoprothesen, erleichtert werden, da eine automatisierte Berechnung der Einstellparameter erfolgt, die zusätzlich an das individuelle Versorgungsoptimum der jeweils versorgten Person angepasst werden kann. Zusätzlich kann durch die Rückmeldung in der Ausgabeeinrichtung 4 eine Dokumentation der Steuersignale sowie eine Empfehlung zu einer verbesserten Nutzung der orthopädietechnischen Einrichtung oder gegebenenfalls Trainingshinweise für den Nutzer gegeben werden.

In dem Computer 3 werden aus den Steuersignalen 5 die gewünschten Parameter für die jeweilige Funktion errechnet. Dabei werden die jeweils von dem Patienten oder Nutzer möglichen und tatsächlich ausgeführten Umschaltsignale oder Steuersignale identifiziert und die optimierten Parameter für die jeweiligen Funktionen werden festgelegt. Es wird also das von einem Nutzer mögliche Steuersignal, was von ihm bereitgestellt werden kann, erfasst und ausgewertet und auf die notwendigen oder möglichen Funktionen der orthopädietechnischen Einrichtung 1 abgebildet, so dass eine Parametereinstellung für die Umschaltvorgänge zwischen den vorhandenen oder möglichen Funktionen vorgeschlagen und individuell nach der Ausgabe in der Ausgabeeinrichtung 4 angepasst werden kann. So können beispielsweise Schwellwerte, Zeitfenster oder Verstärkungsfaktoren der Signale automatisiert ausgewählt und eingestellt werden.

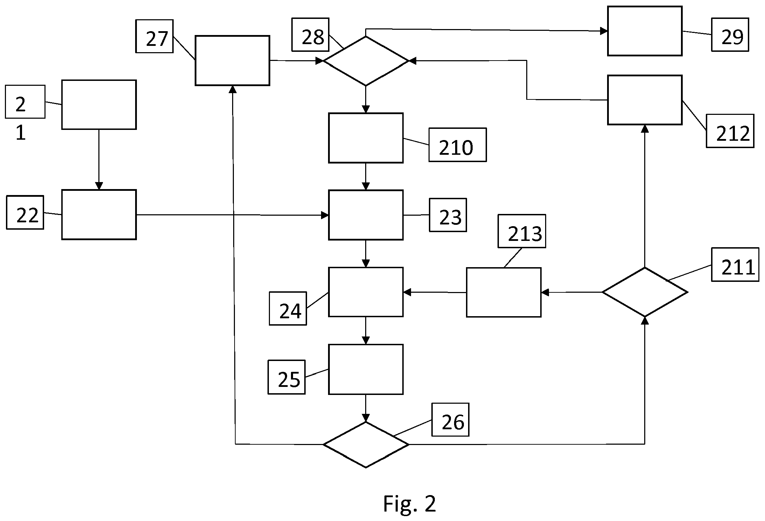

Figur 2 zeigt einen möglichen Ablauf eines Verfahrens zum Einstellen einer Steuereinrichtung für eine orthopädietechnische Einrichtung mit einem ersten Schritt 21. Im ersten Schritt 21 wird ein Anwender identifiziert, gegebenenfalls werden Nutzerdaten des Anwenders aufgenommen und gespeichert und dem jeweiligen Prozess zugeordnet. Darüber hinaus wird eine Anleitung durch einen Aufnahmeprozess zur Sammlung aller relevanten Bewegungsmuster dem Anwender zur Verfügung gestellt. In dem Computer ist beispielsweise die Gesamtheit aller möglichen Funktionen hinterlegt, die die orthopädietechnische Einrichtung ausführen kann. Um diese Funktionen den jeweiligen Steuersignalen des Patienten zuordnen zu können, werden in einzelnen Programmschritten die jeweiligen Funktionen über die Ausgabeeinrichtung 4 abgefragt. Der Nutzer muss dann die entsprechenden Bewegungen sozusagen ausführen, zumindest die Muskelkontraktionen oder Bewegung willentlich ausführen wollen, damit dann über die Sensoren 5 die Steuersignale erfasst werden können. Da diese Steuersignale eine gewisse Qualität aufweisen müssen, können diese mit Verstärkungsfaktoren beaufschlagt werden, über die die Signalstärke vergrößert oder verringert werden kann. Darüber hinaus muss überprüft werden, ob die erfassten Steuersignale in einem Parameterbereich liegen, der eine Auswertung überhaupt ermöglicht. Die einzelnen Programme werden in der Regel mehrfach durchlaufen, um eine ausreichend hohe Wiederholgenauigkeit sicherstellen zu können.

Im zweiten Schritt 22 wird nach der Einführung mit dem ersten Programm begonnen. Zunächst wird im Schritt 23 der größtmögliche Verstärkungsfaktor für die Steuersignale von den Sensoren 5 gewählt.

Im Schritt 24 werden die gesammelten Anwenderdaten mit dem aktuell gewählten, größtmöglichen Verstärkungsfaktor verstärkt. Anschließend wird in dem Schritt 25 für die verstärkten Anwenderdaten alle für das gegenwärtig absolvierte Programm relevanten Parameterwerte berechnet, beispielsweise sind dies die Signaldauer, die Signalamplitude, das Erreichen von Schwellwerten, das Überschreiten von Schwellwerten.

In dem nächsten Schritt 26 wird abgefragt, ob alle relevanten Parameter, die für die jeweilige Funktion notwendig sind, in dem jeweils dafür zulässigen Wertebereich liegen. Ist dies der Fall, wird in einem Schritt 27 das Programm als funktionsfähig gespeichert und der aktuelle Verstärkungsfaktor wird als zugehöriger, optimaler Verstärkungsfaktor gespeichert. Nach der Speicherung als optimaler Verstärkungsfaktor wird in dem nächsten Schritt 28 überprüft, ob das gerade aktuelle Programm das letzte zu überprüfende Programm gewesen ist, also ob alle möglichen Funktionen für die Einstellung dieser orthopädietechnischen Einrichtung 1 an diesem Patienten abgefragt worden sind. Ist dies der Fall, wird im Schritt 29 eine Liste aller funktionsfähigen Programme zur Bereitstellung an einer Ausgabeeinrichtung 4 und gegebenenfalls zur Überprüfung durch einen Orthopädietechniker oder einer anderen Person bereitgestellt. In dieser Liste oder in der Darstellung aller funktionsfähigen Programme ist es möglich, die jeweiligen Funktionen auszuwählen und gegebenenfalls später zu verändern.

Ergibt die Abfrage im Schritt 28, dass das aktuelle Programm nicht das letzte zu überprüfende Programm gewesen ist, wird im Schritt 210 das nächste Programm in der Liste aller möglichen Programme ausgewählt und dieses Programm dem Arbeitsschritt 23 zugeführt, so dass die nächsten Schritte wie oben beschrieben ablaufen.

Wird in dem Arbeitsschritt 26 festgestellt, dass nicht alle relevanten Parameter in einem zulässigen Bereich liegen, wird in einem Schritt 211 abgefragt, ob der aktuelle Verstärkungsfaktor der kleinstmögliche Verstärkungsfaktor war. Wird dies bejaht, gibt es also keinen kleineren Verstärkungsfaktor, wird das ausgeführte Programm in einem Schritt 212 als nicht funktionsfähig gespeichert. Die durch den Patienten erreiche Signalstärke, Signalstaue, Rhythmisierung, Flankensteilheit oder anderen Parametern sind also nicht ausreichend, um ein ausreichend klares Triggersignal zu generieren, damit die Funktion ausgeführt werden kann. Sofern das nicht funktionsfähige Programm das letzte zu überprüfende gewesen ist, wird gemäß Schritt 28 eine Liste mit allen funktionsfähigen Programmen im Schritt 29 generiert. Ist das aktuelle Programm nicht das letzte zu überprüfende Programm gewesen, wird gemäß Schritt 211 das nächste Programm in der Liste gewählt und die Schritte 23 bis 26 durchlaufen.

Ist in dem Verfahrensschritt 211 bei der Abfrage, ob der aktuelle Verstärkungsfaktor der kleinstmögliche war, eine Verringerung des Verstärkungsfaktors möglich, also war der aktuelle Verstärkungsfaktor nicht der kleinstmögliche Verstärkungsfaktor, wird in dem Schritt 213 der nächst kleinere Verstärkungsfaktor gewählt und der Programmablauf mit dem Schritt 24 erneut durchgeführt, nämlich indem die Anwenderdaten mit dem aktuell gewählten, also dem nächstkleineren Verstärkungsfaktor verstärkt wird und anschließend die relevanten Parameterwerte für das aktuelle Programm mit den so verstärkten Anwenderdaten berechnet.

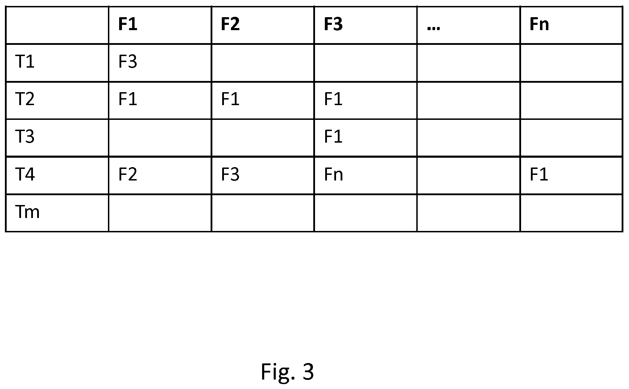

Figur 3 zeigt das Ergebnis eines vollständigen Programmablaufes mit allen möglichen Funktionen der orthopädietechnischen Einrichtung 1, bei der die Triggersignale oder Steuersignale T in der linken Spalte aufgetragen sind und die Funktionen F in der ersten Zeile aufgetragen sind. Es sind Triggersignale T1 bis Tm möglich, als Funktionen stehen die Funktionen F1 bis Fn zur Verfügung. Als Beispiele für Triggersignale mit Sensoren 5 als zwei Elektrodenpaare zur Bereitstellung einer Zwei-Kanal-Versorgung wäre beispielsweise ein kurzes Signal auf zwei Kanälen gleichzeitig, ein langes Signal auf zwei Kanälen gleichzeitig, ein kurzer Impuls auf dem ersten Kanal, einer kurzen Impuls auf dem zweiten Kanal, ein Zeitschalter oder ein schnelles Signal, was insbesondere bei einer Vier-Kanal-Steuerung einsetzbar ist. Als Beispiele für Funktionen F bei einer orthopädietechnischen Einrichtung 1 in Gestalt einer Handprothese dienen der Oppositionsgriff, der Lateralgriff, eine Hook-Funktion, eine Handgelenksflexion, eine Handgelenksextension oder eine Handgelenksrotation.

Mit unterschiedlichen Triggersignalen T1 bis Tm lassen sich unterschiedliche Funktionen F1 bis Fn erreichen. Anhand der beispielhaften Schaltmatrix ist die fertiggestellte Zuordnung nach einem Programmdurchlauf gezeigt. Der Matrix ist zu entnehmen, welche Funktion F nach dem Einleiten eines Triggersignals T erzielt oder eingeleitet werden kann, wenn man von einer bestimmten Funktion F ausgeht. Wenn man sich beispielsweise in der Funktion F1 befindet und das Triggersignal T1 ausführt, gelangt man zu der Funktion F3. Wenn man sich in der Funktion F3 befindet und das Triggersignal T2 ausführt, gelangt man zu der Funktion F1. Unabhängig von der Funktion, in der man sich gerade befindet, gelangt man durch die Aktivierung des Triggersignals T2 immer zu der Funktion F1. Durch die Ausführung des Triggersignals T4 wird immer eine Funktion weiter geschaltet, also ausgehend von der Funktion F1 gelangt man durch Aktivierung des Triggersignals T4 zur Funktion F2, ausgehend von der Funktion F2 gelangt man zu der Funktion F3 nach dem Ausführen des Triggersignals T4 usw. Mit dem Triggersignal T4 ist es somit möglich, alle Funktionen der Reihe nach durchzuschalten.

Ist eine solche Matrix erstellt worden, nachdem die Steuersignale ausgewertet worden sind, kann z.B. ein Orthopädietechniker erkennen, welche Funktion der Prothese von dem speziellen Patienten überhaupt angesteuert werden kann, um diese dann mit einem gesonderten Aktivierungssignal auszuführen. Auch der Patient kann der Matrix entnehmen, welches Steuersignal oder Triggersignal am besten geeignet ist, um die vorhandenen Funktionen anzusteuern und anschließend zu aktivieren.

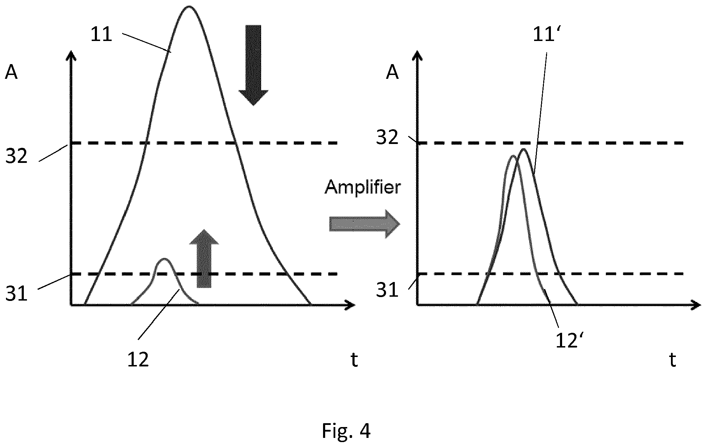

Figur 4 zeigt in der linken Darstellung ein zweikanaliges Steuersignal einer Co-Kontraktion mit einem ersten Signal 11 und einem zweiten Signal 12. Das erste Signal 11 weist eine wesentlich größere Amplitude A und eine längere Signaldauer T als das zweite Signal 12 auf. Das erste Signal 11 überschreitet einen oberen Grenzwert 32, das zweite Signal 12 überschreitet einen unteren Grenzwert 31. Nach der Aufforderung an den Patienten, eine bestimmte Bewegung auszuführen, ergibt sich in dem Beispiel der Figur 4 eine längere und stärkere Kontraktion des einen Muskels, was zu dem ersten Signal 11 führt, während ein kokontrahierter Muskel kürzer und schwächer angespannt wird, so dass das zweite Signal 12, ein myoelektrisches Signal, entsprechend kürzer und mit einer geringeren Amplitude ausfällt. Um ein Triggersignal T oder ein Umschaltsignal zum Umschalten in eine andere Funktion für die Steuerungseinheit 7 sicher bereitstellen zu können, sollten beide Signale 11, 12 zwischen den beiden Schwellwerten 31, 32 liegen. Bei der Signalauswertung wird über einen Algorithmus detektiert, dass das erste Signal 11 gedämpft werden muss, also mit einem negativen Verstärkungsfaktor versehen werden muss, wohingegen das zweite Signal 12 verstärkt werden muss, also mit einem positiven Verstärkungsfaktor beaufschlagt werden muss. Beide Signale 11, 12 sollten möglichst nahe an dem oberen Schwellwert 32 liegen, um eine ausreichende Signalstärke und damit eine ausreichende Identifizierbarkeit zu gewährleisten. Dementsprechend wird über einen Verstärker oder eine Verstärkungsfunktion das erste Signal 11 gedämpft und hinsichtlich der Zeitdauer komprimiert, während das zweite Signal 12 hinsichtlich der Amplitude verstärkt wird. Die Zeitdauer des zweiten Signals 12 bleibt unverändert, das erste Signal 11 wird hinsichtlich des Beginns auf den Beginn des zweiten Signals 12 verschoben. Das Ergebnis ist in der rechten Darstellung der Figur 4 gezeigt, bei der die optimierten Signale 11', 12'immer noch ihre charakteristische Form aufweisen, jedoch hinsichtlich ihrer Amplitude und ihrer Dauer verändert sind. Bei der Verstärkung ist darauf zu achten, dass alle anderen Triggersignale oder Steuersignale ebenfalls funktionieren, so dass ein gemeinsamer Verstärkungsfaktor hinsichtlich der Amplitude und die Zeitverschiebung und -verkürzung hinsichtlich der Zeitdauer des Signals auf alle Steuersignale gleichmäßig angewendet werden kann.

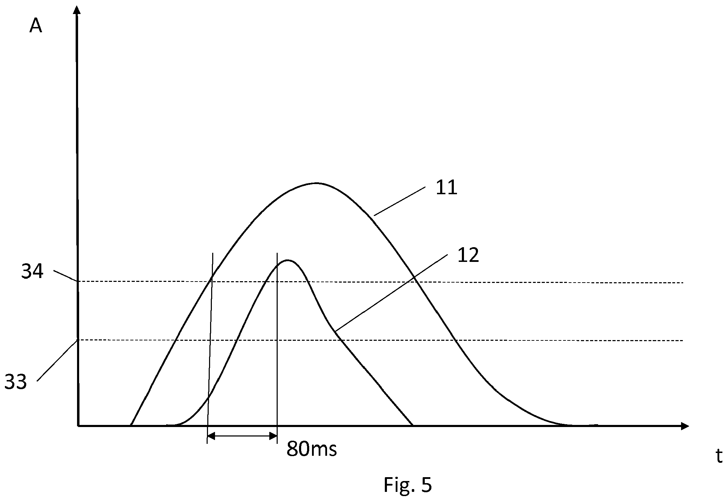

Figur 5 zeigt eine beispielhafte Bedingung, die erfüllt sein muss, damit ein zweikanaliges Steuersignal mit einem ersten Signal 11 und einem zweiten Signal 12 überhaupt ausgewertet und verstärkt werden kann. Als Bedingung ist vorgesehen, dass innerhalb einer bestimmten Zeitspanne beide Signale 11, 12 einen Einschaltgrenzwert 34 überschreiten müssen. Der Einschaltgrenzwert 34 bezieht sich auf die Signalamplitude, in dem dargestellten Ausführungsbeispiel erreicht das erste Signal 11 den Einschaltschwellwert 34, der oberhalb eines Ausschaltgrenzwertes 33 liegt, zuerst und wird dann innerhalb einer Zeitspanne von ca. 50 ms auch von dem zweiten Signal 12 erreicht. Das Erreichen des Einschaltgrenzwertes 34 durch das zweite Signal 12 liegt innerhalb der vorgegebenen Zeitspanne, im dargestellten Ausführungsbeispiel von 80 ms, so dass die erste Voraussetzung für die Verwertung des aufgenommenen Signals gegeben ist und beide Signale 11, 12 eine zunächst ausreichende Signalqualität aufweisen.

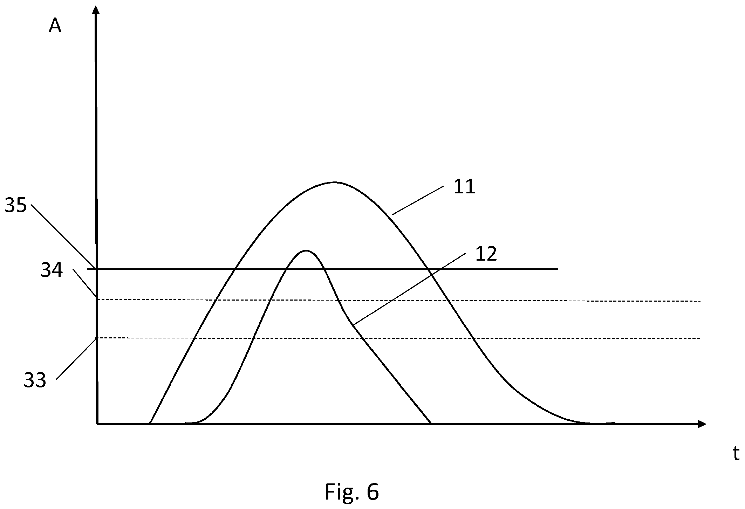

Figur 6 zeigt eine weitere Anforderung an beide Signale 11, 12, um als Triggersignal T für die Einleitung oder Umschaltung zwischen einzelnen Funktionen F zu dienen. Beide Signale 11, 12 müssen einen Kokontraktionsschwellwert 35 überschreiten, also eine gewisse Mindestamplitude erreichen, die oberhalb des Einschaltgrenzwertes 34 liegt. Liegt beispielsweise der Einschaltgrenzwert 34 bei 0,5 V, der Ausschaltgrenzwert bei 0,3 V, kann der Kokontraktionsschwellwert bei 0,6 V liegen.

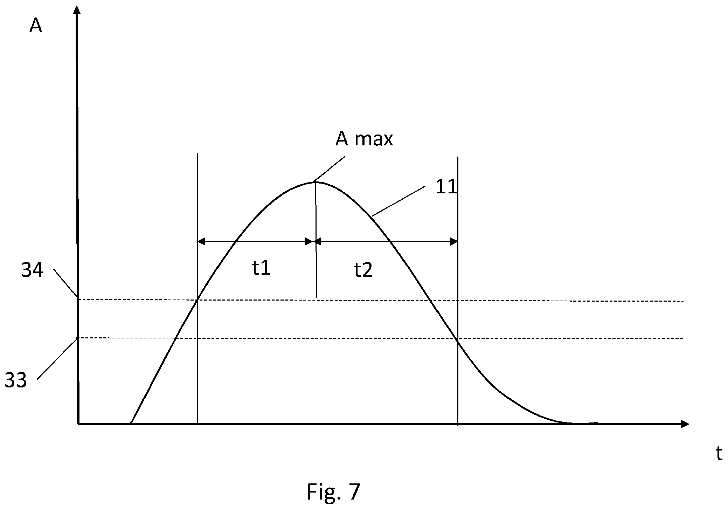

Eine dritte Bedingung für eine ausreichende Signalqualität ist in der Figur 7 dargestellt, bei der vorgegeben ist, dass die Amplitude in einem vorbestimmten Zeitraum T1 vor Erreichen des Amplituden-Maximalwerts Amax und in einem zweiten Zeitraum T2 nach Erreichen der maximalen Amplitude Amax oberhalb eines der beiden Grenzwerte 33, 34 liegen muss. Eine Festlegung kann beispielsweise sein, dass nach Durchlaufen des Einschaltgrenzwertes 34 innerhalb einer gewissen Zeitspanne T1 ein maximaler Amplitudenwert Amax erreichen werden muss und die Signalamplitude nach Erreichen des maximalen Amplitudenwertes Amax über einen zweiten Zeitraum T2 oberhalb des Ausschaltgrenzwertes 33 liegen muss, damit das Kokontraktionssignal, im vorliegenden Fall das erste Signal 11, verwertet werden kann.

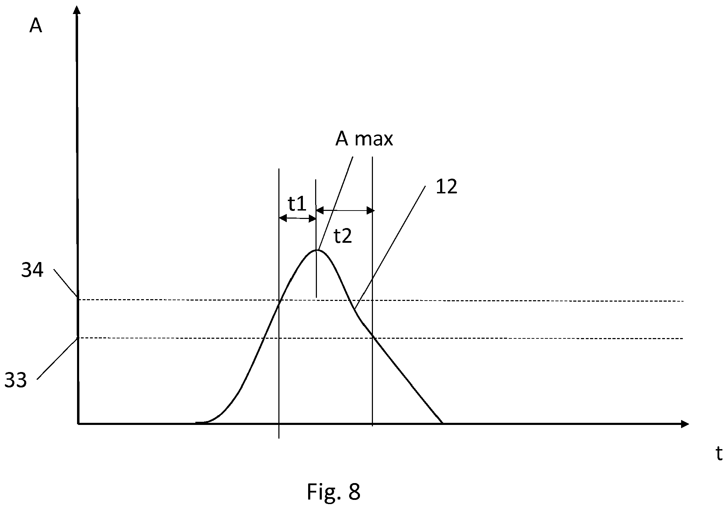

Figur 8 zeigt die korrespondierende Bedingung für das zweite Signal 12, das von einer zweiten Elektrode erfasst wird, während das erste Signal 11 von einer ersten Elektrode oder einem ersten Sensor 5 erfasst wird.

Werden alle Bedingungen erfüllt, kann über den jeweils gewählten Verstärkungsfaktor eine Aufbereitung der empfangenen Steuersignale in dem Computer 3 erfolgen, so dass über die Ausgabeeinrichtung 4 sowohl die erfassten Steuersignale als auch die Verstärkungen und die zugeordneten Funktionen F angezeigt werden können. Die Verstärkungsfaktoren können nachträglich angepasst werden, beispielsweise über eine Benutzeroberfläche auf der Ausgabeeinrichtung 4 des Computers 3. Über die nach der Ausgabe der Bewegungsaufforderung erfassten Steuersignale ist es möglich, die von dem jeweiligen Patienten überhaupt möglichen Signale hinsichtlich ihrer Signalqualität zu erfassen und zu beurteilen und den entsprechenden Bewegungen zuzuordnen. Ist ein Patient nicht in der Lage, eine ausreichende Signalqualität für das Umschalten auf eine bestimmte Funktion oder einen bestimmten vorgegebenen Bewegungsablauf zu erzeugen, kann diese Funktion entweder ausgelassen werden und als nicht aktiviert in der Steuerungseinrichtung 7 abgelegt werden oder aber es wird ein anderer Signalverlauf notwendig oder aber ein anderer Verstärkungsfaktor wird gewählt.

Die Ausgabe der von dem Patienten erzeugten Steuersignale dient zudem der Dokumentation, zu welchen Steuersignalen der Patient überhaupt fähig ist. Lässt die Signalqualität nach, kann beispielsweise ein Patient eine gewisse Signalstärke oder Amplitude A nicht mehr erreichen, kann dies als Indikator dienen, ein Trainingsprogramm aufzulegen oder auch um Trainingsfortschritte zu dokumentieren.

## Patentansprüche

1. Verfahren zum Einrichten einer Steuerung einer orthopädietechnischen Einrichtung mit zumindest einem motorischen Antrieb, die an einem Körperteil eines Patienten angelegt und mit Sensoren verbunden ist, die Steuersignale des Patienten aufnehmen,
mit folgenden Schritten:
Ausgeben einer optischen, akustischen und/oder taktilen Darstellung einer Betätigung einer Gliedmaße als Aufforderung an den Patienten, diese Betätigung auszuführen;
Erfassen von Steuersignalen, die von dem Patienten nach dessen Aufforderung als willkürliche Reaktion erzeugt werden,
Zuordnen der erfassten Steuersignale zu der ausgeführten Betätigung und zu einer Funktion, in der der zumindest eine motorische Antrieb aktiviert, deaktiviert oder in seiner Drehrichtung umgekehrt wird, sowie Ausgeben der erfassten Steuersignale und/oder der Funktion nach der Zuordnung zu der jeweiligen Funktion.

2. Verfahren nach Anspruch 1, **dadurch gekennzeichnet, dass** die Steuersignale über genau zwei Elektroden oder ein Elektrodenpaar aufgenommen werden.

3. Verfahren nach Anspruch 1 oder 2, **dadurch gekennzeichnet, dass** aus den erfassten Steuersignalen Parameter abgeleitet werden, die für die Zuordnung der jeweiligen Funktion relevant sind.

4. Verfahren nach einem der voranstehenden Ansprüche, **dadurch gekennzeichnet, dass** die Bewegungsaufforderung in Form vorgegebener Schaltsignale ausgegeben wird.

5. Verfahren nach einem der voranstehenden Ansprüche, **dadurch gekennzeichnet, dass** eine Zuordnung eines Steuersignals zu einer Funktion auf der Grundlage einer Signalstärke nach Überschreiten eines festlegbaren Schwellwertes erfolgt.

6. Verfahren nach einem der voranstehenden Ansprüche, **dadurch gekennzeichnet, dass** vor der Zuordnung zu einer Funktion eine Bestätigungsabfrage durchgeführt wird.

7. Verfahren nach einem der voranstehenden Ansprüche, **dadurch gekennzeichnet, dass** vor der Zuordnung zu einer Funktion eine Auswertung der Steuersignale hinsichtlich der Signalqualität durchgeführt und bei nicht ausreichender Signalqualität eine Fehlermeldung oder ein Korrekturvorschlag ausgegeben wird.

8. Verfahren nach einem der voranstehenden Ansprüche, **dadurch gekennzeichnet, dass** die Steuersignale vor der Zuordnung mit festgelegten Korrekturfaktoren beaufschlagt werden.

9. Verfahren nach einem der voranstehenden Ansprüche, **dadurch gekennzeichnet, dass** die Steuersignale, die von dem Patienten nach Aufforderung als willkürliche Reaktion erzeugt werden, nach jeder Aufforderung gespeichert werden.

10. Verfahren nach einem der voranstehenden Ansprüche, **dadurch gekennzeichnet, dass** die Steuersignale, die von dem Patienten nach Aufforderung als willkürliche Reaktion erzeugt werden, mit vorgegebenen Zielwerten verglichen und hinsichtlich des Erreichens dieser Zielwerte bewertet werden.

11. System zur Durchführung des Verfahrens nach einem der voranstehenden Ansprüche mit
a. einer an eine Körperteil eines Patienten anlegbaren orthopädietechnischen Einrichtung mit zumindest einem motorischen Antrieb,
b. mit einer Ausgabeeinrichtung, die optische, akustische und/oder taktile Darstellungen einer Betätigung einer Gliedmaße als Aufforderung an den Patienten, diese Betätigung auszuführen, ausgibt,
c. mit Sensoren, die mit der orthopädietechnischen Einrichtung verbunden und an dem Patienten festlegbar sind und Steuersignale des Patienten aufnehmen,
d. mit einer elektronischen Auswerteeinrichtung, in der die Steuersignale, die von dem Patienten nach Aufforderung als willkürliche Reaktion erzeugt werden, verarbeitet, ausgewertet und einer Funktion zugeordnet werden und
e. mit einer Ausgabeeinrichtung, in der die jeweilige, dem Steuersignal zugeordnete Funktion ausgegeben wird.

12. System nach Anspruch 11, **dadurch gekennzeichnet, dass** die Auswerteeinrichtung eine Schnittstelle aufweist, über die die Zuordnung zu der Funktion beeinflussbar ist.

## Claims

1. A method for setting up a controller of an orthopedic device comprising at least one motor drive, which is placed against a body part of a patient and connected to sensors that record control signals of the patient,
including the following steps:
outputting an optical, acoustic, and/or tactile representation of an actuation of a limb as a prompt for the patient to carry out this activity;
capturing control signals produced as a deliberate reaction by the patient after being prompted,
assigning the captured control signals to the activity carried out and to a function, within the scope of which the at least one motor drive is activated, deactivated, or reversed in terms of its direction of rotation, and
outputting the captured control signals and/or the function after the assignment to the respective function.

2. The method as claimed in claim 1, **characterized in that** the control signals are recorded by way of exactly two electrodes or one electrode pair.

3. The method as claimed in claim 1 or 2, **characterized in that** parameters that are relevant to the assignment of the respective function are derived from the captured control signals.

4. The method as claimed in anyone of the preceding claims, **characterized in that** the prompt to move is output in the form of predefined switching signals.

5. The method as claimed in anyone of the preceding claims, **characterized in that** a control signal is assigned to a function on the basis of a signal strength once a predefinable threshold has been exceeded.

6. The method as claimed in anyone of the preceding claims, **characterized in that** a confirmation is requested before the assignment to a function.

7. The method as claimed in anyone of the preceding claims, **characterized in that** the control signals are evaluated in respect of the signal quality before the assignment to a function and an error message or a correction suggestion is output in the case of an insufficient signal quality.

8. The method as claimed in anyone of the preceding claims, **characterized in that** predefined correction factors are applied to the control signals before the assignment.

9. The method as claimed in any one of the preceding claims, characterized that the control signals produced by the patient as a deliberate reaction following the prompt are stored after every prompt.

10. The method as claimed in anyone of the preceding claims, **characterized in that** the control signals produced by the patient as a deliberate reaction following the prompt are compared to predefined target values and assessed in respect of attaining these target values.

11. A system for carrying out the method as claimed in anyone of the preceding claims, comprising
a. an orthopedic device which is able to be placed against a body part of a patient and comprises at least one motor drive,
b. comprising an output device which outputs optical, acoustic and/or tactile representations of an actuation of a limb as a prompt for the patient to carry out this activity,
c. comprising sensors which are connected to the orthopedic device, able to be fastened to the patient, and record control signals of the patient,
d. comprising an electronic evaluation device in which the control signals produced by the patient as a deliberate reaction following the prompt are processed, evaluated, and assigned to a function, and
e. comprising an output device, in which the respective function assigned to the control signal is output.

12. The system as claimed in claim 11, **characterized in that** the evaluation device comprises an interface, by means of which the assignment to the function is able to be influenced.

## Revendications

1. Procédé de configuration d'une commande d'un dispositif orthopédique qui comprend au moins un entraînement motorisé et qui est appliqué sur une partie du corps d'un patient et qui est relié à des capteurs recevant des signaux de commande du patient,
comprenant les étapes suivantes consistant à :
émettre une représentation optique, acoustique et/ou tactile d'un actionnement d'un membre comme invitation au patient à effectuer cet actionnement ;
détecter les signaux de commande générés par le patient à titre de réaction arbitraire en réponse à l'invitation de ce dernier,
associer les signaux de commande détectés à l'actionnement effectué et à une fonction dans laquelle ledit au moins un entraînement motorisé est activé, désactivé ou dont le sens de rotation est inversé, et
émettre les signaux de commande détectés et/ou la fonction selon l'association à la fonction respective.

2. Procédé selon la revendication 1,
**caractérisé en ce que** les signaux de commande sont enregistrés par l'intermédiaire de précisément deux électrodes ou d'une paire d'électrodes.

3. Procédé selon la revendication 1 ou 2,
**caractérisé en ce que** des paramètres pertinents pour l'association de la fonction respective sont déduits des signaux de commande détectés.

4. Procédé selon l'une des revendications précédentes,
**caractérisé en ce que** l'invitation au mouvement est émise sous la forme de signaux de commutation prédéfinis.

5. Procédé selon l'une des revendications précédentes,
**caractérisé en ce qu'**une association d'un signal de commande à une fonction est effectuée sur la base d'une intensité de signal après dépassement d'une valeur seuil définissable.

6. Procédé selon l'une des revendications précédentes,
**caractérisé en ce qu'**une demande de confirmation est effectuée avant l'association à une fonction.

7. Procédé selon l'une des revendications précédentes,
**caractérisé en ce que**, avant l'association à une fonction, une évaluation des signaux de commande est effectuée en ce qui concerne la qualité des signaux et, si la qualité des signaux n'est pas suffisante, un message d'erreur ou une proposition de correction est émis(e).

8. Procédé selon l'une des revendications précédentes,
**caractérisé en ce que**, avant l'association, les signaux de commande sont soumis à des facteurs de correction définis.

9. Procédé selon l'une des revendications précédentes,
**caractérisé en ce que** les signaux de commande générés par le patient à titre de réaction arbitraire en réponse à une invitation sont enregistrés après chaque invitation.

10. Procédé selon l'une des revendications précédentes,
**caractérisé en ce que** les signaux de commande générés par le patient à titre de réaction arbitraire en réponse à une invitation sont comparés à des valeurs cibles prédéfinies et sont évalués quant à savoir si ces valeurs cibles sont atteintes.

11. Système pour la mise en oeuvre du procédé selon l'une des revendications précédentes, comprenant
a. un dispositif orthopédique pouvant être appliqué sur une partie du corps d'un patient et comportant au moins un entraînement motorisé,
b. un dispositif de sortie qui émet des représentations optiques, acoustiques et/ou tactiles d'un actionnement d'un membre comme invitation au patient à effectuer cet actionnement,
c. des capteurs qui sont reliés au dispositif orthopédique et peuvent être fixés au patient et qui enregistrent des signaux de commande du patient,
d. un dispositif d'évaluation électronique dans lequel les signaux de commande, générés par le patient à titre de réaction arbitraire en réponse à l'invitation, sont traités, évalués et associés à une fonction, et
e. un dispositif de sortie dans lequel la fonction respective associée au signal de commande est émise.

12. Système selon la revendication 11,
**caractérisé en ce que** le dispositif d'évaluation présente une interface par laquelle l'association à la fonction peut être influencée.
